# EUROPÄISCHE PATENTSCHRIFT

(11) **EP 0 945 120 B1**
(45) Veröffentlichungstag und Bekanntmachung des Hinweises auf die Patenterteilung: **26.11.2003**
(21) Anmeldenummer: 99101739.3
(22) Anmeldetag: 11.02.1999
(51) Int. Cl.: A61K 7/032

(54) **Mascara und Augenbrauenstifte enthaltend Gamma-Oryzanol und Calciumsalze**
Mascara and eyebrowpencils containing gamma-oryzanol and calcium salts
Mascara et crayons pour sourcils contenant du gamma-oryzanol et des sels de calcium

(30) Priorität: 11.02.1998 DE 19805428
(43) Veröffentlichungstag der Anmeldung: 29.09.1999
(73) Patentinhaber: Beiersdorf Aktiengesellschaft, 20245 Hamburg (DE)
(72) Erfinder: Riedel, Jan-Henric, Dr., 21075 Hamburg (DE); Petsitis, Xenia, 65719 Hofheim A.T. (DE)

(56) Entgegenhaltungen:
- DE-A- 1 617 557
- FR-A- 1 145 887
- DATABASE WPI Week 9622 Derwent Publications Ltd., London, GB; AN 96-217163 XP002101734 & JP 08 081352 A (OKAYASU SHOTEN KK)
- THE MERCK INDEX, TWELFTH EDITION: "Oryzanol (p.1181, paragraph 7016)", 1996, MERCK RESEARCH LABORATORIES, WHITEHOUSE STATION, NJ

## Beschreibung

Die vorliegende Erfindung betrifft kosmetische Wirkstoffkombinationen und Zubereitungen, solche Wirkstoffkombinationen enthaltend. Insbesondere betrifft die vorliegende Erfindung Mascara und Augenbrauenstifte mit einem Gehalt an Substanzen, die die Wimpern oder die Augenbrauen und die diese umgebende Haut, aber auch die Zubereitungen selbst vor unerwünschten Oxidationsprozessen schützen. In einer bevorzugten Ausführungsform betrifft die vorliegende Erfindung Wirkstoffkombinationen und Zubereitungen damit, die dazu dienen, solche Haare und die Haut in deren Umgebung zu pflegen.

Oxidative Prozesse schädigen Stoffe unterschiedlichster Natur (z.B. Haut, Haare und Wolle, aber auch Lacke und Kunststoffe, um nur einige zu nennen) zum Teil in erheblichem Maße. Die Stoffe ändern dabei ihre physikalischen und chemischen Eigenschaften: sie "altern". Zur Verzögerung oder sogar Inhibierung der Alterung von Stoffen werden im allgemeinen sogenannte Alterungsschutzmittel verwendet.

Insbesondere die schädigende Wirkung des ultravioletten Teils der Sonnenstrahlung auf organische, aber auch anorganische Stoffe ist allgemein bekannt. Für den Menschen sind dabei die Schädigung von Haut und Haaren und die Verzögerung oder Verhinderung derselben durch den Einsatz von Lichtschutzmitteln von besonderer Bedeutung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares und der Wimpern und Augenbrauen dürfte allerdings heute darin bestehen, das Aussehen des Menschen in charakteristischer Weise mitzugestalten. Ähnlich wie die Haut erfüllen sie eine soziale Funktion, da sie über ihr Erscheinungsbild erheblich zu zwischenmenschlichen Beziehungen und zum Selbstwertgefühl des Individuums beitragen.

Die Haare, d.h. auch die Augenbrauen und Wimpern, bestehen aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die ein ganzes Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluß des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluß auf das Haar, der sich beispielsweise darin äußert, daß bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Haut Teile des Körpers dar, die aufgrund ihrer Position beim Aufenthalt im Freien einer erheblichen Menge an UV-Strahlung ausgesetzt sind.

Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Gewöhnlich werden dabei Wasserstoffperoxidkonzentrationen um z.B. 3% verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagiert und dabei unter Umständen eine Schädigung des Haares bewirken kann.

Oxidative Einflüsse, wie beispielsweise chemische Haarbehandlungen oder Sonnenlicht, setzen demnach die Festigkeit und die Elastizität des Haares herab und führen zu einer Zerstörung von Melanin. Augenscheinlich wird dies insbesondere bei dunkelhaarigen Personen, deren Haar im Sommer durch das intensive Sonnenlicht deutlich aufgehellt wird. Unter dem Bergriff "oxidativer Einfluß" ist im Sinne der vorliegenden Erfindung sowohl der Einfluß von oxidierend wirkenden Substanzen zu verstehen als auch die oxidative Wirkung von durch Strahlung, namentlich Licht, insbesondere UV-Licht, hervorgerufenen Folgeprodukten.

Ein Ziel der Pflege von Haaren ist es, sie vor oxidativen Einflüssen zu schützen und den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für ein natürliches, gesundes Haar.

Die Verwendung von Antioxidantien, also Substanzen, die Oxidationsprozesse verhindern, in Kosmetika ist an sich bekannt. Antioxidantien, die in der Kosmetik Verwendung finden, sind beispielsweise Tocopherole, Gallensäurederivate, Sesamol und Flavonoide. Antioxidantien werden hauptsächlich als Schutzsubstanzen gegen der Verderb der sie enthaltenden Zubereitungen verwendet.

Auch Tocopherole, insbesondere Vitamin E, sind natürlich prinzipiell geeignet, Oxidationsprozesse zu verhindern, und finden dementsprechend häufig in Kosmetika Verwendung. Allerdings haben Tocopherole den Nachteil, daß sie im allgemeinen sehr reaktiv sind und daher zu einem beträchtlichen Teil bereits in der Zubereitung abreagieren. Dies führt dazu, daß nur ein kleiner Teil der Einsatzmenge den zu schützenden Körperteil überhaupt erreicht, so daß die erzielte Wirkung weit hinter der erhofften zurückbleibt.

Augen Make-up, insbesondere Mascaraprodukte (Wimpemtusche), haben im dekorativen Marktsegment eine große Bedeutung. Mit Mascara kann man die Wimpern optisch effektvoll betonen und den Augen dadurch mehr Ausdruck verleihen. Auch mit Augenbrauenstiften kann man die Augenpartie ausdrucksvoll betonen.
Die Qualität einer Mascara läßt sich messen an einem guten und leichten Auftrag, einer angemessenen Trockenzeit und einer guten Haftfestigkeit. Weiterhin sollte eine Mascara sich nicht nachteilig auf das Wimpernhaar auswirken, z.B. nicht austrocknend wirken oder die Brüchigkeit erhöhen. Im Gegenteil: Es ist wünschenswert, eine Mascara zu erhalten, die neben den genannten Aspekten sogar noch pflegende Eigenschaften aufweist. Dieser Pflegeaspekt wurde bei der Entwicklung von Wimperntusche in der Vergangenheit auf Kosten des optischen Effekts nicht berücksichtigt.

Aufgabe der vorliegenden Erfindung war, die Nachteile des Standes der Technik zu beseitigen. Insbesondere sollten kosmetische Wirkstoffe bzw. Zubereitungen, solche Wirkstoffe enthaltend, zur Verfügung gestellt werden, bei deren Verwendung die Schädigung von Wimpern und Augenbrauen durch oxidativen Einfluß gemindert, wenn nicht gänzlich verhindert werden kann.

Erfindungsgemäß wird diese Aufgabe gelöst und werden die Nachteile des Standes der Technik beseitigt.

Gegenstand der Erfindung sind Mascara und Augenbrauenstifte, dadurch gekennzeichnet, daß sie Gamma-Oryzanol und ein Calciumsalz oder mehrere Calciumsalze, ausgewählt aus der Gruppe, gebildet von Calciumpanthotenat, Calciumchlorid und Calciumlactat, enthalten.

Gegenstand der Erfindung sind auch dekorative haarkosmetische Wirkstoffkombinationen, dadurch gekennzeichnet, daß sie Gamma-Oryzanol und ein Calciumsalz oder mehrere Calciumsalze, ausgewählt aus der Gruppe, gebildet von Calciumpanthotenat, Calciumchlorid und Calciumlactat, enthalten.

Gegenstand der Erfindung ist weiterhin die Verwendung von Wirkstoffkombinationen und Mascara und Augenbrauenstiften, die diese enthalten, bestehend aus Gamma-Oryzanol und einem Calciumsalz oder mehreren Calciumsalzen, ausgewählt aus der Gruppe, gebildet von Calciumpanthotenat, Calciumchlorid und Calciumlactat, zum Schutz haarkosmetischer Zubereitungen und/oder der Wimpern und Augenbrauen vor unerwünschten Oxidationsprozessen.

Bevorzugte haarkosmetische Zubereitungen sind Mascara und Augenbrauenstifte.

Die erfindungsgemäßen Wirkstoffkombinationen und Zubereitungen, diese Wirkstoffkombinationen enthaltend, mindern die Schädigung der Haut und/oder des Haares durch oxidative Einflüsse besser als Wirkstoffe, Wirkstoffkombinationen und Zubereitungen des Standes der Technik. Insbesondere pflegen sie durch oxidativen Streß geschädigte oder strapazierte Haare bzw. beugen solchen Schäden vor.

Gamma-Oryzanol ist in der Literatur beschrieben (CAS-Nr.: 11042-64-1 bzw. 12738-23-7). Gamma-Oryzanol ist keine einheitliche Verbindung, sondern ein Gemisch verschieden strukturierter Ferulasäureester. Gamma-Oryzanol besteht hauptsächlich aus den Estern der Ferulasäure mit den Triterpenalkoholen Cycloartenol und 24-Methylencylcloartenol. Ferner enthält es geringe Mengen an Estern mit Sterolen, namentlich mit Camesterol, Stigmasterol und β-Sitosterol.

Die anti-oxidative Aktivität des Gamma-Oryzanols in Modellen des Cytochrom-System wird im Merck Index (12. Ausgabe, 1996) erwähnt.

Gamma-Oryzanol ist im Handel erhältlich, beispielsweise unter dem Handelsnamen Gamma-Oryzanol® (Lieferant: Jan Dekker oder Henry Lamotte, DE).

Vorteilhaft enthalten erfindungsgemäße Zubereitungen 0,001 bis 10,0 Gew.-% Gamma-Oryzanol, bevorzugt 0,05 bis 5,0 Gew.-%, jeweils bezogen auf das Gesamtgewicht der Zubereitungen.

Vorteilhaft enthalten erfindungsgemäße Zubereitungen 0,01 bis 25 Gew.-% eines oder mehrerer Calciumsalze, bevorzugt 0,1 bis 5 Gew.-%, jeweils bezogen auf das Gesamtgewicht der Zubereitungen.

Als Calciumsalz wird Calciumpanthotenat bevorzugt. Die Calciumsalze sind im Handel erhältlich.

Die Zubereitungen, die erfindungsgemäße Wirkstoffkombinationen enthalten, sind topische Zubereitungen. Diese können wie üblich zusammengesetzt sein und zur Behandlung und der Pflege der Wimpern und Augenbrauen oder der die umgebenden Haut dienen. Zur Anwendung werden die erfindungsgemäßen Zubereitungen in der üblichen Weise in ausreichender Menge aufgebracht.

Aus DE 16 17 557 ist eine Zubereitung zur Schönheitspflege bekannt, die Gamma-Oryzanol und Calcium-pantothenat enthält. Es handelt sich dabei aber um eine wässrig-alkoholische Nährlösung, die nicht zur Verwendung in Mascara und Augenbrauenstiften geeignet ist.

Vorteilhaft können Zubereitungen im Sinne der vorliegenden Erfindung beispielsweise als Blockmascara, Mascara in Cremeform (wasserfrei oder emulgiert), oder als Augenbrauenstifte, z.B. als "Crayons" oder "Pencil" vorliegen.

Die erfindungsgemäßen Wirkstoffkombinationen können in allen üblichen Wimperntuschen, Mascara und Augenbrauenstift-Zubereitungen verwendet werden.

Vorteilhaft enthalten erfindungsgemäße Zubereitungen neben einem wirksamen Gehalt an erfindungsgemäßen Wirkstoffkombinationen ferner übliche Farb-, Wirk-, Inhalts-, Zusatzund/oder Hilfsstoffe.

Präparate vom "Cake"-Typ (Block-Mascara) bestehen aus einer Mischung von Farbstoff, Fetten und Wachsen und Öl-in-Wasser-Emulgatoren. Als Emulgator verwendet man zumeist Triäthanolaminstearat.
Beim Auftragen des Präparates verwendet man eine feuchte Bürste. Durch das anwesende Wasser bildet sich an der Oberfläche des Mascarablockes eine Öl-in-Wasser-Emulsion. Diese wird von der Bürste aufgenommen. Auf den Wimpern trocknet sie wieder ein.

Wasserfreie Mascara sind überwiegend gelförmige Zubereitungen auf Öl-Wachsbasis mit einem hohen Anteil flüchtiger Lösungsmittel.

Wasserhaltige, emulgierte Mascara in Cremeform besitzen als Basis z.B. eine Öl-in-Wasser-Creme vom Stearat- oder Glycerylmonostearattyp.

Bevorzugte Emulsions-Mascara im Sinne der vorliegenden Erfindung enthalten als Hauptbestandteile beispielsweise synthetische und/oder natürliche Wachse,synthetische und/oder natürliche Öle, wie z.B. Triglyceride der Linolsäure, O/W-Emulgatoren, vorzugsweise Stearinsäure, neutralisiert mit TEA oder NaOH, und ggf. einen Coemulgator, einen oder mehrere wasserlösliche Filmbildner, vorzugsweise aus der Klasse der PVP/VA-Copolymere oder Ammoniumacrylate, mehrwertige Alkohole, vorzugsweise Butandiol oder Propandiol, Pigmente und/oder Perlglanzpigmente (Eisenoxide, Ultramarinblau, Chromhydroxydgrün), Füllstoffe, vorzugsweise Talkum, Kaolin oder Mica und gegebenenfalls auch Hilfsstoffe.

Bevorzugte wasserfeste Mascara enthalten beispielsweise Gelbildner, z.B. Quarternium-18-hectorite (Bentone-38®), Aktivator für den Gelbildner, z.B. Alkohole wie z.B. Ethanol, Verdicker, z.B. substituierte und unsubstituierte Cellulosen, Wachse zur Strukturgebung, öl- bzw. fettlösliche Filmbildner, z.B. PVP/Eicosene-Copolymer, Pigmente, wie z.B. vorstehend angegeben, Lösungsmittel, z.B. Kohlenwasserstoffe, vorzugsweise Kettenlänge C9-C12 und/oder flüchtige Silikone, z.B. Cyclomethicone und gegebenenfalls Hilfsstoffe.

Viele dekorative Augenprodukte, wie z.B. Augenbrauenfärbemittel, werden in Form von Stiften angeboten. Zum Nachzeichnen und Colorieren der Augenbrauen werden meist Formulierungen verwendet, bei denen die farbgebenden Pigmente in einer Wachs-/Öl-Basis eingelagert sind. Im allgemeinen wird die fettige Farbstoff-Wachsmasse als Mine extrudiert und in Stiften aus Rotzedernholz konfektioniert.

Augenbrauenstifte werden heute in zwei Formen verwendet: als gegossene Stifte und als "Pencils". Die Schminkstifte sind, dem Aufbau nach, harte Fettschminken. Geeignete Augenbrauenstifte des Standes der Technik mit einem Gehalt an Paraffinen und Bienenwachs bzw. Japanwachs sind in "Kosmetik, Entwicklung, Herstellung und Anwendung kosmetischer Mittel", Herausgeber: W. Umbach, Georg Thieme Verlag, Stuttgart-New York, 1995, S. 321 ff, beschrieben.

Die erfindungsgemäßen Zubereitungen können kosmetische Hauptkomponenten und Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hauptkomponenten beträgt beispielsweise 85 bis 99,999 Gew.-%, vorzugsweise 90 bis 99,99 Gew.-%, jeweils bezogen auf das Gesamtgewicht der Zubereitung.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die erfindungsgemäßen Zubereitungen können Wasser, z.B. 1 bis 80 Gew.-% und/oder Alkohole, z.B. 0,5 bis 10 Gew.-% enthalten, jeweils bezogen auf das Gesamtgewicht der Zubereitungen.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 10 Gew.-%, vorzugsweise 0,05 bis 5 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zweckmäßigerweise sind Komplexbildner, insbesondere Komplexierungsmittel oder Bestandteile der Zubereitungen, die in dieser Weise, oder Calciumionen bindend wirken, nicht oder nur in geringer Menge in den Zubereitungen enthalten. Durch zusätzliche Dosierung von Ca-Ionen oder höhere Anteile der Calciumsalze in der erfindungsgemäßen Kombination können aber diese Einwirkungen gegebenenfalls auch, wie dem Fachmann bekannt, ausgeglichen werden. Vorzugsweise liegt der Anteil von Calciumbindemitteln, falls diese gewünscht sind, z.B. unter 0,001 Gew.-% bis 0,01 Gew.-%, insbesondere aber unter 0,1 Gew.-%, jeweils bezogen auf das Gewicht der Zubereitungen.

Das Gewichtsverhältnis der Wirkstoffe Oryzanol/Calciumsalze zueinander in den Kombinationen kann in einem breiten Bereich variiert werden und kann beispielsweise 100 : 1 bis 1 : 100, vorzugsweise 10 : 1 bis 1 : 10 und insbesondere aber auch 1:1 betragen.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.

Als Gamma-Oryzanol wird in den Beispielen das Handelsprodukt Gamma-Oryzanol® der Firma Jan Dekker, DE, verwendet.

In den folgenden Beispielen werden als Vinylpyrrolidon-Vinylacetat-Copolymer z B. Luviskol VA 37 E® (BASF) verwendet und als PVP/Eisosene Copolymer z.B. Unimer U15® (Induchem AG) verwendet.

Die Mengenangaben in den Beispielen sind Gew.-%.

| **Beispiele 1 bis 3** | | | |
|---|---|---|---|
| Emulsionsmascara | | | |
| Stearinsäure | 3,50 | 4,00 | 4,00 |
| Ozokerit | 7,00 | 6,00 | 5,00 |
| Camaubawachs | 3,00 | 3,50 | 3,50 |
| Neutralöl | 3,00 | 4,00 | - |
| Oleyl Alcohol | - | - | 3,00 |
| Sorbitansesquioleat | 1,50 | 1, 80 | 1, 80 |
| Triethanolamin | 1,00 | 1,10 | 1,10 |
| Polyvinylpyrrolidon | 7,50 | - | 7,50 |
| Vinylpyrrolidon-Vinylacetat-Copolymer | - | 7,00 | - |
| Butandiol | 2,00 | 1,50 | 1,40 |
| Talkum | 5,00 | 3,00 | - |
| Farbpigmente (Eisenoxide) | 8,00 | 9,00 | 10,00 |
| Calciumpanthotenat | 1,30 | 4,80 | 0,10 |
| Gamma-Oryzanol | 2,30 | 0,20 | 5,00 |
| Konservierungsmittel | q.s. | q.s. | q.s. |
| Wasser, demineralisiert | ad 100 | ad 100 | ad 100 |

| **Beispiele 4 und 5** | | |
|---|---|---|
| "Waterproof Mascara" | | |
| Quarternium-18-Hectorite | 10,00 | 12,00 |
| Talkum | 5,00 | 4,00 |
| Ethanol | 2,50 | 2,75 |
| Hydroxypropylcellulose | 1,00 | 0,50 |
| Carnaubawachs | 2,00 | - |
| PVP/Eicosene Copolymer | 20,00 | 18,00 |
| Perlglanzpigmente | 5,00 | 3,00 |
| Farbpigmente (Eisenoxide) | 10,00 | 10,00 |
| Nylon-6 | - | 2,00 |
| Cyclomethicone | 5,00 | - |
| Konservierungsmittel | q.s. | q.s. |
| Calciumpanthotenat | 0,80 | 0,10 |
| Gamma-Oryzanol | 0,20 | 5,00 |
| Isoparaffin C10-12 | ad 100 | ad 100 |

## Patentansprüche

1. Mascara und Augenbrauenstifte, **dadurch gekennzeichnet, daß** sie Gamma-Oryzanol und ein Calciumsalz oder mehrere Calciumsalze, ausgewählt aus der Gruppe, gebildet von Calciumpanthotenat, Calciumchlorid und Calciumlactat, enthalten.

2. Dekorative haarkosmetische Wirkstoffkombinationen, **dadurch gekennzeichnet, daß** sie Gamma-Oryzanol und ein Calciumsalz oder mehrere Calciumsalze, ausgewählt aus der Gruppe, gebildet von Calciumpanthotenat, Calciumchlorid und Calciumlactat, enthalten.

3. Verwendung von Wirkstoffkombinationen in Mascara und Augenbrauenstiften, die diese enthalten, bestehend aus Gamma-Oryzanol und einem Calciumsalz oder mehreren Calciumsalzen, ausgewählt aus der Gruppe, gebildet von Calciumpanthotenat, Calciumchlorid und Calciumlactat, zum Schutz haarkosmetischer Zubereitungen und/oder der Wimpern und Augenbrauen vor unerwünschten Oxidationsprozessen.

4. Zubereitungen nach Anspruch 1, **dadurch gekennzeichnet, daß** zusätzlich weitere Tenside und/oder kosmetische oder dermatologische Hilfs-, Zusatz- und/oder Wirkstoffe enthalten sind.

5. Zubereitungen nach Anspruch 1, **dadurch gekennzeichnet, daß** der Gehalt an Oryzanol 0,001 bis 10,0 Gew.-%, bezogen auf das Gesamtgewicht der Zubereitungen, beträgt.

6. Zubereitungen nach Anspruch 1, **dadurch gekennzeichnet, daß** der Gehalt an einem oder mehreren Calciumsalzen 0,01 bis 25 Gew.-%, bevorzugt 0,02 bis 5 Gew.-%, jeweils bezogen auf das Gesamtgewicht der Zubereitungen, beträgt.

7. Verwendung nach Anspruch 3, **dadurch gekennzeichnet, daß** der Gehalt an Oryzanol 0,001 bis 10,0 Gew.-%, bezogen auf das Gesamtgewicht der Zubereitungen, beträgt.

8. Verwendung nach Anspruch 3, **dadurch gekennzeichnet, daß** der Gehalt an einem oder mehreren Calciumsalzen 0,01 bis 25 Gew.-%, bevorzugt 0,02 bis 5 Gew.-%, jeweils bezogen auf das Gesamtgewicht der Zubereitung, beträgt.

## Claims

1. Mascara and eyebrow sticks, **characterized in that** they comprise gamma-oryzanol and one or more calcium salts chosen from the group formed by calcium panthotenate [sic], calcium chloride and calcium lactate.

2. Decorative hair cosmetic active ingredient combinations, **characterized in that** they comprise gamma-oryzanol and one or more calcium salts chosen from the group formed by calcium panthotenate [sic], calcium chloride and calcium lactate.

3. Use of active ingredient combinations in mascara and eyebrow sticks which comprise these, consisting of gamma-oryzanol and one or more calcium salts chosen from the group formed by calcium panthotenate [sic], calcium chloride and calcium lactate, for protecting hair cosmetic preparations and/or eyelashes and eyebrows against undesired oxidation processes.

4. Preparations according to Claim 1, **characterized in that** further surfactants and/or cosmetic or dermatological auxiliaries, additives and/or active ingredients are additionally present.

5. Preparations according to Claim 1, **characterized in that** the content of oryzanol is 0.001 to 10.0% by weight, based on the total weight of the preparations.

6. Preparations according to Claim 1, **characterized in that** the content of one or more calcium salts is 0.01 to 25% by weight, preferably 0.02 to 5% by weight, in each case based on the total weight of the preparations.

7. Use according to Claim 3, **characterized in that** the content of oryzanol is 0.001 to 10.0% by weight, based on the total weight of the preparations.

8. Use according to Claim 3, **characterized in that** the content of one or more calcium salts is 0.01 to 25% by weight, preferably 0.02 to 5% by weight, in each case based on the total weight of the preparation.

## Revendications

1. Mascara et crayons pour sourcils, **caractérisés en ce qu'**ils contiennent du gamma-oryzanol et un sel calcique ou plusieurs sels calciques choisis dans le groupe constitué du pantothénate de calcium, du chlorure de calcium et du lactate de calcium.

2. Combinaisons de substances actives cosmétiques de maquillage capillaire, **caractérisées en ce qu'**elles contiennent du gamma-oryzanol et un sel calcique ou plusieurs sels calciques choisis dans le groupe constitué du pantothénate de calcium, du chlorure de calcium et du lactate de calcium.

3. Utilisation de combinaisons de substances actives dans du mascara et des crayons pour sourcils, qui les contiennent, constituées de gamma-oryzanol et d'un sel calcique ou de plusieurs sels calciques choisis dans le groupe constitué du pantothénate de calcium, du chlorure de calcium et du lactate de calcium, pour la protection de compositions cosmétiques capillaires et/ou des cils et des sourcils contre des processus d'oxydation non souhaités.

4. Compositions selon la revendication 1, **caractérisées en ce qu'**elles contiennent en outre d'autres agents tensioactifs et/ou adjuvants, additifs et/ou substances actives cosmétiques ou dermatologiques.

5. Compositions selon la revendication 1, **caractérisées en ce que** la teneur en oryzanol est de 0,001 à 10,0% en poids par rapport au poids total des compositions.

6. Compositions selon la revendication 1, **caractérisées en ce que** la teneur en un ou plusieurs sels calciques est de 0,01 à 25% en poids, de préférence de 0,02 à 5% en poids, à chaque fois par rapport au poids total des compositions.

7. Utilisation selon la revendication 3, **caractérisée en ce que** la teneur en oryzanol est de 0,001 à 10,0% en poids par rapport au poids total des compositions.

8. Utilisation selon la revendication 3, **caractérisée en ce que** la teneur en un ou plusieurs sels calciques est de 0,01 à 25% en poids, de préférence de 0,02 à 5% en poids, à chaque fois par rapport au poids total des compositions.
